# EUROPÄISCHE PATENTSCHRIFT

(11) **EP 3 941 367 B1**
(45) Veröffentlichungstag und Bekanntmachung des Hinweises auf die Patenterteilung: **07.12.2022**
(21) Anmeldenummer: 20708480.7
(22) Anmeldetag: 03.03.2020
(51) Int. Cl.: A61B 17/28, A61B 17/3201, A61B 90/00, A61B 17/00

(54) **VERFAHREN ZUR EINFACHEN HERSTELLUNG EINER REINIGUNGSOPTIMIERTEN INSTRUMENTENFEDER**
METHOD FOR SIMPLE PRODUCTION OF AN INSTRUMENT SPRING OPTIMIZED IN TERMS OF CLEANING
PROCÉDÉ POUR LA PRODUCTION SIMPLE D'UN RESSORT D'INSTRUMENT OPTIMISÉ POUR LE NETTOYAGE

(30) Priorität: 18.03.2019 DE 102019106852
(43) Veröffentlichungstag der Anmeldung: 26.01.2022
(73) Patentinhaber: Aesculap AG, 78532 Tuttlingen (DE)
(72) Erfinder: VOGTHERR, Robert, 78532 Tuttlingen (DE)
(74) Vertreter: Winter, Brandl - Partnerschaft mbB
(86) Internationale Anmeldenummer: PCT/EP2020/055486
(87) Internationale Veröffentlichungsnummer: WO 2020/187553

(56) Entgegenhaltungen:
- DE-A1-102014 102 606
- DE-A1-102017 114 260
- DE-U-202011 052 256
- US-A1- 2003 083 747
- US-A1- 2005 222 588

## Beschreibung

### Technisches Gebiet

Die vorliegende Erfindung betrifft ein Verfahren zur Herstellung eines medizinischen Handinstruments gemäß dem Oberbegriff des Anspruchs 1, insbesondere ein Verfahren zur Herstellung eines chirurgischen Handinstrument der Zangen- oder Scherenbauart, mit zwei Griffelementen oder Griffbügeln, welche relativ zueinander schwenkbar sind, und einem Federelement, vorzugsweise einer Blattfeder, die U- oder V-förmig gebogen ist, welches zwei Federelementenden aufweist, die jeweils mit einem der beiden Griffelemente verbunden sind, so dass bei Verschwenken zumindest eines der beiden Griffelemente aus einer Grundstellung ein Zurückschwenken in die Grundstellung mittels des Federelements bewerkstelligbar ist.

### Stand der Technik

Die DE 10 2017 114 260 A1 zeigt ein medizinisches Handinstrument mit zwei Griffelementen, welche relativ zueinander schwenkbar sind, und einem Federelement, welches zwei Federelementenden aufweist. Die Federelementenden sind jeweils mit einem der beiden Griffelemente verbunden, so dass bei Verschwenken zumindest eines der beiden Griffelemente aus einer Grundstellung ein Zurückschwenken in die Grundstellung mittels des Federelements bewerkstelligbar ist. Das Handinstrument zeichnet sich dabei dadurch aus, dass zumindest eines der beiden Federelementenden mit dem entsprechenden Griffelement über einen Formschluss verbunden ist, der durch Umformen des Federelements erzeugt ist. Des Weiteren offenbart die DE 10 2017 114 260 A1 ein Verfahren zum Herstellen eines medizinischen Handinstruments.

In der Druckschrift DE 20 2010 007 995 U1 wird ein Instrument offenbart, bei welchem eine einteilige Blattfeder auf einer Seite des Instruments bzw. Instrumentengriffes verschraubt ist und mit ihrem freien Ende auf der gegenüberliegenden Seite des Instruments bzw. am anderen Instrumentengriff anliegt. Dies birgt etliche Nachteile: Generell sollte bei chirurgischen Instrumenten so gut als möglich auf Gewinde verzichtet werden, da die Spalte zwischen Mutterngewinde und Schraube quasi nicht reinigbar sind. Bei dem Instrument gemäß Druckschrift DE 20 2010 007 995 U1 liegt die Blattfeder im Bereich der Verschraubung direkt an der Innenseite des Instruments an, wodurch sich auch hier ein sehr enger, nicht reinigbarer Spalt zwischen Blattfeder und Instrument ergibt. Feuchtigkeit oder Reinigungsflüssigkeit kann aus diesem Spalt sehr schlecht austrocknen und kann über einen gewissen Zeitraum korrosiv wirken. Zudem ist die Blattfeder in diesem korrosionsanfälligen Bereich durchbohrt und in ihrer Geometrie geschwächt, wodurch die Blattfeder an dieser Stelle bruchgefährdet ist. Im Auflagebereich auf der gegenüberliegenden Instrumentenseite reibt das freie Blattfeder-Ende während der Bewegung bzw. Benutzung des Instruments. Diese Reibung verschlechtert auf Dauer die Oberflächengüte des Auflagebereichs und vergrößert dort ebenfalls die Korrosionsanfälligkeit.

In der Druckschrift DE 20 2009 002 433 U1 ist ein Instrument mit zwei Griffbranchen offenbart, bei welchem pro Griffbranche jeweils ein angeschraubtes Blattfederteil vorgesehen ist. Um ein Zurückschwenken der beiden Griffbranchen in eine Grundstellung zu bewirken, stützen sich die beiden Blattfederteile aneinander ab. Hinsichtlich seiner Reinigbarkeit weist das Instrument gemäß Druckschrift DE 20 2009 002 433 U1 insbesondere wegen seinen Verschraubungen die gleichen Nachteile wie das Instrument gemäß Druckschrift DE 20 2010 007 995 U1 auf. Weiterhin besteht im Kontaktbereich der beiden freien Blattfederenden eine Steckverbindung, die relativ scharfkantig ist. Da sich diese Stelle mitten im gut zugänglichen Griffbereich des Instruments befindet, kann ein OP-Handschuh des Anwenders hier leicht eingeschnitten oder beschädigt werden. Und auch wenn diese Verbindungsstelle im Neuzustand des Instruments gut entgratet ist, wird sich nach etlichen Anwendungen ein gewisser Abrieb und Grat bilden.

Bei den beiden vorstehend genannten Druckschriften DE 20 2010 007 995 U1 und DE 20 2009 002 433 U1 zeigen sich auch im Hinblick auf die Herstellung aufwändig und anfällig. Die Schraube zum Befestigen der Feder wird durch die Bohrung in der Blattfeder hindurch in das Gewinde im Griff geschraubt. Bei diesem Arbeitsschritt ist es wichtig, die Schraube mit ausreichendem Drehmoment anzuziehen, damit sie sich nicht durch Transport, Erschütterungen oder Instrumentenbewegungen löst. Gerade bei Verschmutzung und einer damit einhergehenden Schwergängigkeit des Gewindes ist dies nicht sichergestellt. Des Weiteren ist der Federmechanismus einfach austauschbar, aber in gleichem Maße einfach manipulierbar. Ein Anwender kann die Schraube für eine Reinigung einfach lösen. Im Anschluss an die Reinigung ist jedoch nicht sichergestellt, dass er die Schraube auch wieder ordnungsgemäß befestigt. So kann zudem die Schraube verloren gehen und Störungen im klinischen Ablauf verursachen.

Die Druckschrift DE 20 2009 001 809 U1 bezieht sich auf ein Instrument, dessen Federmechanismus wie bei dem Instrument gemäß Druckschrift DE 20 2009 002 433 U1 aus zwei verschraubten Blattfederteilen besteht. Um zumindest die genannten Nachteile einer scharfkantigen Steck-Verbindungsstelle zu vermeiden, ist zwischen den Blattfederteilen eine Kugel- und Pfannengeometrie vorgesehen. Aufgrund der Verschraubungen und der Kugel- und Pfannengeometrie ist das Instrument gemäß DE 20 2009 001 809 U1 hinsichtlich seiner Reinigbarkeit aber weiterhin nachteilhaft. Darüber hinaus ist der Federmechanismus gemäß DE 20 2009 001 809 U1 aufwändig herzustellen, da die dargestellten Geometrien an den freien Blattfederenden angeschweißt oder komplex gefräst werden müssen.

Die Druckschrift DE 20 2011 052 256 U1 beschreibt ein Instrument, dessen Federmechanismus aus einem verschraubten Blattfederteil und einem an dessen freien Ende gelenkig gelagert angehängten zweiten Teil besteht. Aufgrund der Verschraubung und der Komplexität des Federmechanismus ist auch das Instrument gemäß Druckschrift DE 20 2011 052 256 U1 verhältnismäßig schlecht reinigbar und aufwendig herzustellen.

Instrumente, bei welchen die Federmechanismen in aufwendiger Art zumindest teilweise integral mit entsprechenden Griffteilen ausgebildet sind, werden beispielsweise in den Druckschriften DE 101 37 915 B4, DE 10 2007 030 874 B4 oder DE 10 2014 102 606 A1 beschrieben. Weitere Beispiele solcher Instrumente sind in den Druckschriften US 2005 / 222 588 A1 oder US 2003 / 083 747 A1 offenbart. Diese Instrumente sind nicht nur aufgrund ihrer aufwändigen Herstellung, sondern auch aufgrund der schlechten Ersetz- bzw. Demontierbarkeit der Blattfederteile nachteilig.

### Offenbarung der Erfindung

In Anbetracht der Herstellverfahren der Instrumente gemäß dem genannten Stand der Technik ist es Aufgabe der vorliegenden Erfindung, ein kosten- und zeitsparendes Verfahren zur Herstellung eines gut reinigbaren, sich verhältnismäßig schwach abnutzenden und/oder relativ einfach zu reparierenden medizinischen Handinstruments bereitzustellen.

Diese Aufgabe wird durch ein Verfahren zur Herstellung eines medizinischen Handinstruments mit den Schritten des Anspruchs 1 gelöst. Vorteilhafte Weiterbildungen sind Gegenstand der Unteransprüche.

Die vorliegende Erfindung betrifft folglich ein Verfahren zur Herstellung eines medizinischen Handinstruments mit zwei relativ zueinander schwenkbaren Griffelementen/Griffbranchen (Griffbügeln, Griffhebeln, Hebelarmen) und einem, vorzugsweise U- oder V-förmig gebogenen, insbesondere in Blattfederbauart ausgeführten, Federelement (Vorspannfeder). Jedes der beiden Enden des Federelements (Federelementenden) wird im Anschluss daran jeweils mit einem entsprechenden der beiden Griffelemente verbunden. Danach werden die beiden Griffelemente derart miteinander verbunden, dass bei Verschwenken zumindest eines der beiden Griffelemente aus einer Grundstellung relativ zu dem anderen Griffelement ein Zurückschwenken in die Grundstellung mittels des Federelements bewerkstelligbar ist.

In anderen Worten ausgedrückt sieht das vorliegende Verfahren zur Herstellung eines medizinischen Handinstruments u.a. die folgenden Verfahrensschritte in der angegebenen Reihenfolge vor:
a) Bereitstellen von zwei vorzugsweise baugleichen (Instrumenten-)Griffelementen jeweils mit einer daran ausgebildeten Schwenkzapfen-Aufnahmeöse, an deren einem Endabschnitt und einer ausgebildeten, weiter vorzugsweise in deren Mittenabschnitt daran geschlitzten, Anlenköse für ein Federelement, vorzugsweise der Blattfederbauart,
b) Bereitstellen des Federelements mit an jedem Federende jeweils angeordneten oder vorzugsweise stoffeinstückig mit dem Federelement ausgebildeten Schwenkzapfen,
c) Einsetzen der Schwenkzapfen des Federelements in die Anlenkösen der noch nicht miteinander schwenkgekoppelten Instrumenten-Griffelemente und
d) Zusammenführen der Instrumenten-Griffelemente im Bereich von deren Schwenkzapfen-Aufnahmeösen und Schwenkkoppeln der Instrumenten-Griffelemente durch Einführen eines vorzugsweise niet- oder schraubenförmigen Schwenkzapfens in die sich überlappenden/fluchtenden Schwenkzapfen-Aufnahmeösen.

Durch dieses Verfahren wird die Montage des medizinischen Handinstruments vereinfacht und die Instrumenteneigenschaften, insbesondere die Federvorspannung reproduzierbar.

Das erfindungsgemäße Verfahren zur Herstellung des medizinischen Instruments zeichnet sich insbesondere dadurch aus, dass das Einsetzen der Schwenkzapfen des Federelements in die Anlenkösen vor dem Zusammenführen und Schwenkkoppeln der Griffelemente durchgeführt wird. Mit anderen Worten, ist bzw. wird das Federelement so ausgebildet, dass es sich an dem entsprechenden Griffelement einhaken kann, ohne vor, beim oder nach dem Einhaken umgeformt werden zu müssen.

In einer bevorzugten Ausgestaltung kann somit die formschlüssige Verbindung der beiden Federelementenden mit den entsprechenden Griffelementen durch das Verbinden der beiden Griffelemente gesichert sein. Somit ist eine sichere Handhabe des medizinischen Handinstruments gewährleistet, ohne einen zusätzlichen Arbeitsschritt zu benötigen.

Vorzugsweise können darüber hinaus die beiden Griffelemente formschlüssig verbindbar und lösbar sein, so dass das medizinische Handinstrument zur Reinigung oder Reparatur einfach durch Lösen der Griffelement-Verbindung demontiert werden kann.

Erfindungsgemäß kann in dem Herstellverfahren die Anlenköse/Aufnahme so ausgebildet sein, dass diese das entsprechende Federelementende formschlüssig nach einem Schlüssel-Schloss-Prinzip aufnimmt. Dabei können die Aufnahmen im Wesentlichen ringförmig ausgestaltet sein und jeweils einen Schlitzabschnitt aufweisen, durch welchen das entsprechende Federelementende in die zugehörige Aufnahme einführbar ist. Ferner können an den Federelementenden jeweils zwei sich gegenüberliegende Aussparungen ausgebildet sein, welche nach dem Einführen der Federelementenden in die Aufnahme diese umgreifen.

Mit anderen Worten ausgedrückt, können an den Federelementenden jeweils zwei sich gegenüberliegende Aussparungen ausgebildet sein, so dass die Federelementenden als Schwenkzapfen jeweils einen T-förmigen Endabschnitt aufweisen. Der Querbalken des T-förmigen Endabschnitts kann dabei jeweils als eine Schwenkachse dienen.

### Kurzbeschreibung der Zeichnungen

Die vorliegende Erfindung wird im Folgenden anhand eines bevorzugten Ausführungsbeispiels unter Bezugnahme auf die beigefügten Zeichnungen näher beschrieben. Es zeigen:
Figur 1 eine perspektivische Detailansicht eines mit einem erfindungsgemäßen Verfahren hergestellten, medizinischen Handinstruments gemäß einer bevorzugten Ausführungsform;
Figur 2 eine perspektivische Detailansicht der Verhakung des Federelementendes mit dem Griffelement gemäß der bevorzugten Ausführungsform; und
Figur 3 eine Draufsicht des Federelementendes gemäß der bevorzugten Ausführungsform.

Gleiche oder funktional äquivalente Merkmale sind in den einzelnen Figuren mit denselben Bezugszeichen versehen.

### Detaillierte Beschreibung einer bevorzugten Ausführungsform

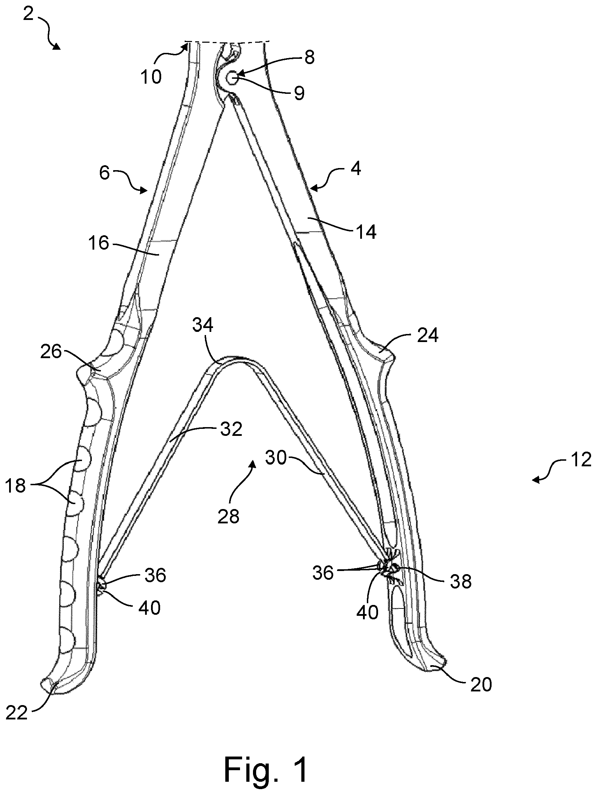

Figur 1 zeigt ein medizinisches Handinstrument 2 gemäß einer bevorzugten Ausführungsform. Das Handinstrument 2 ist nach Zangen- oder Scherenbauart ausgebildet. Das heißt, es weist zwei Hebel/Instrumentenbranchen 4 und 6 auf, welche miteinander über ein Scharnier 8 schwenkbar verbunden sind. Die Hebel 4 und 6 weisen dafür jeweils im Bereich des Scharniers 8 eine (Schwenkzapfen-)Aufnahmeöse auf, welche zur Montage des medizinischen Handinstruments 2 so zusammengeführt werden, dass ihre Mittelachsen im Wesentlichen fluchten. Wie nachstehend beschrieben, wird im Anschluss an das Zusammenführen der Hebel 4 und 6 eine Niete 9 durch die Aufnahmeösen geführt und so werden die beiden Hebel 4 und 6 über das Scharnier 8 miteinander verbunden. Die Niete 9 ist hier ein Beispiel für einen "Schwenkzapfen":
Der Abschnitt des Handinstruments 2 einerseits (distale Seite) des Scharniers 8 ist ein (nur ansatzweise gezeigter) Maulabschnitt 10. Der Abschnitt andererseits (proximale Seite) des Scharniers 8 ist ein Griffabschnitt 12. Die proximalen Teile der Hebel 4 und 6, welche den Griffabschnitt 12 des Handinstruments 2 bilden, werden nachfolgend Griffelemente 14 und 16 genannt.

Das Griffelement 14 des Hebels 4 und das Griffelement 16 des Hebels 6 sind im Wesentlichen symmetrisch zueinander ausgebildet. Beide Griffelemente 14 und 16 weisen jeweils an der Seite, welche vom jeweils anderen Griffelement 16 und 14 wegweist, an eine menschliche Hand angepasste Vertiefungen 18 auf, um eine gute Haptik beim Greifen des Handinstruments 2 zu gewährleisten. An der proximalen Seite des Griffabschnitts 12, an den freien Enden der Griffelemente 14 und 16, weisen die Griffelemente 14 und 16 an der Seite, welche vom jeweils anderen Griffelement 16 und 14 wegweist, Griffvorsprünge 20 und 22 auf. Auch jeweils eine Handbreit entfernt von den freien Enden weisen die Griffelemente 14 und 16 an der Seite, welche vom jeweils anderen Griffelement 16 und 14 wegweist, Griffvorsprünge 24 und 26 auf. Die Griffvorsprünge 20 und 24 beziehungsweise 22 und 26 grenzen jeweils einen Bereich an den Griffelementen 14 und 16 ein, an welchen ein Anwender den Griffabschnitt 12 bevorzugt ergreift bzw. bevorzugt ergreifen soll. Die Griffvorsprünge 20 und 24 beziehungsweise 22 und 26 sind dabei dafür bestimmt, ein Abrutschen der Finger des Anwenders zu verhindern.

Zwischen den Griffelementen 14 und 16 ist ein Federelement 28 vorgesehen. Das Federelement 28 ist im Wesentlichen U- bzw. V-förmig und weist zwei Schenkel 30 und 32 auf, welche miteinander über einen bogenförmigen Abschnitt 34 verbunden sind.

Die beiden Schenkel 30 und 32 sowie der bogenförmige Abschnitt 34 sind einstückig in Form einer gebogenen Blattfeder bevorzugt aus Federstahl ausgebildet.

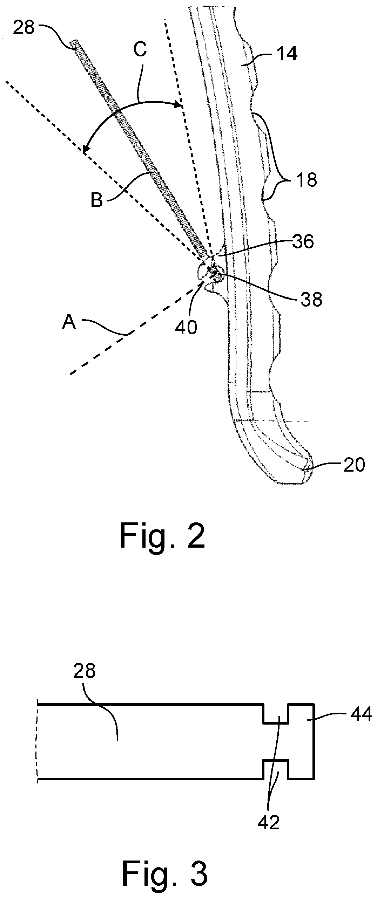

In den Figuren 2 und 3 ist detailliert die Verhakung eines der beiden Federelementenden mit dem entsprechenden Griffelement 14, 16 und die Ausgestaltung des Federelementendes bei dem erfindungsgemäß hergestellten medizinischen Handinstrument 2 gezeigt. Zu beachten ist, dass Figur 2 keine Schnittdarstellung ist, sondern die Schraffierung des Federelements 28 vielmehr der besseren Übersichtlichkeit dient. Auch ist in Figur 2 lediglich die Verbindung zwischen dem Schenkel 30 des Federelements 28 und dem Griffelement 14 gezeigt. Es ist jedoch selbstverständlich, dass die Verbindung zwischen dem Schenkel 32 und dem Griffelement 16 gleich ausgeführt ist.

Das Griffelement 14 weist an der zum anderen (nicht gezeigten) Griffelement 16 weisenden Seite zwei sich in Richtung des anderen Griffelements 16 erstreckende Vorsprünge/Aufnahmen/Anlenkösen 36 auf, welche sich parallel zur Erstreckungsrichtung des Griffelements 14 erstrecken. An ihrem jeweiligen freien Ende weisen die Aufnahmen 36 jeweils ein Lagerauge 38 mit jeweils einem sich zum Rand der jeweiligen Aufnahme 36 erstreckenden Schlitz 40 auf. Die Weite des Schlitzes 40 ist nur unwesentlich größer als die Stärke des Federelements 28.

Ein Verbindungsabschnitt des federblattförmigen Federelements 28 weist, wie in Figur 3 gezeigt, zwei Aussparungen/Ausstanzungen 42 auf, welche derart angeordnet sind, dass ein T-förmiger Endabschnitt 44 am Federelement 28 ausgebildet ist. Anders ausgedrückt weist der Verbindungsabschnitt zwei Vorsprünge auf, welche derart angeordnet sind, dass der T-förmige Endabschnitt 44 gebildet wird. Der Querbalken des T-förmigen Endabschnitts 44 dient dem Federelement 28 im montierten Zustand als Schwenkachse. Der T-förmige Endabschnitt 44 ist somit hier ein Beispiel für einen "Schwenkzapfen" des Federelements 28.

Zum Verhaken des Federelements 28 mit dem entsprechenden Griffelement 14 gemäß der bevorzugten Ausführungsform wird der Querbalken des T-förmigen Endabschnitts 44 durch den Schlitz 40 in das Lagerauge 38 eingeschoben. Der Schenkel 30 des Federelements 28 ist bei diesem Einschieben zwangsläufig parallel zum Schlitz 40 auszurichten (siehe angedeutete Montagestellung A in Figur 2). Gleiches ist bei dem anderen (nicht gezeigten) Griffelement 16 zu tun, um das Federelement 28 zu montieren.

Um schließlich das Federelement 28 mit den Griffelementen 14, 16 fest zu verhaken bzw. zu verrasten, werden die beiden Griffelemente 14, 16 an dem Scharnier 8 miteinander verbunden. In dem Herstellverfahren gemäß der bevorzugten Ausführungsform werden die beiden Griffelement 14, 16 an dem Scharnier 8 miteinander vernietet. Diese Verbindung kann jedoch auch beliebig anders, z.B. als Verschraubung, ausgeführt sein.

Mit anderen Worten, wird das Federelement 28 erfindungsgemäß zunächst mit einem Federelementende des jeweiligen Schenkels 30, 32 in der Aufnahme des entsprechenden Griffelements 14, 16 verhakt und im Anschluss daran wird diese Verhakung durch Verbinden der beiden Griffelemente 14, 16 an dem Scharnier 8 mit der Niete 9 fixiert und gesichert.

Der Umstand, dass der Querbalken in den Lageraugen 38 gefangen ist und nicht mehr durch den Schlitz 40 hindurchbewegt werden kann (siehe Grundstellung B in Figur 2), bleibt solange erhalten, bis die Schenkel 30, 32 des Federelements 28 nicht absichtlich in die Montagestellung A verschwenkt werden. Ein solches Verschwenken der Schenkel 30, 32 des Federelements 28 in die Montagestellung A kann jedoch nur bei mutwilligem Verbiegen des Federelements 28 oder beim Lösen der Verbindung zwischen den Griffelementen 14, 16 auftreten.

Insbesondere in einem normalen Schwenkbereich C (siehe Figur 2), in welchem sich die Schenkel 30, 32 des Federelements 28 beim Gebrauchen des Handinstruments 2 bewegen, bleiben die Endabschnitte des Federelements 28 in den Aufnahmen 36 bzw. den Lageraugen 38 der Griffelemente 14, 16 gefangen.

Die in den Figuren 1 bis 3 gezeigte und oben beschriebene Ausführungsform des mit dem erfindungsgemäßen Verfahren hergestellten medizinischen Handinstruments ist lediglich ein Beispiel einer möglichen Umsetzung der beanspruchten Erfindung.

## Patentansprüche

1. Verfahren zum Herstellen eines medizinischen Handinstruments (2), welches zwei, vorzugsweise baugleiche, relativ zueinander schwenkbare Griffelemente (14, 16) und ein, vorzugsweise nach Blattfederbauart ausgebildetes, Federelement (28) aufweist, mit den Verfahrensschritten:
- Bereitstellen der zwei Griffelemente (14, 16) jeweils mit einer daran ausgebildeten Schwenkzapfen-Aufnahmeöse an deren einem Endabschnitt und einer daran ausgebildeten, vorzugsweise in deren Mittenabschnitt geschlitzten, Anlenköse (36) für das Federelement (28),
- Bereitstellen des Federelements (28) mit jeweils an jedem Federende angeordneten oder, vorzugsweise stoffeinstückig, mit dem Federelement (28) ausgebildeten Schwenkzapfen (44),
- Einsetzen der Schwenkzapfen (44) des Federelements (28) in die Anlenkösen (36) der noch nicht miteinander schwenkgekoppelten Griffelemente (14, 16) und
- Zusammenführen der Griffelemente (14, 16) im Bereich von deren Schwenkzapfen-Aufnahmeösen und Schwenkkoppeln der Griffelemente (14, 16) durch Einführen eines, vorzugsweise niet- oder schraubenförmigen, Schwenkzapfens (9) in die sich überlappenden Schwenkzapfen-Aufnahmeösen.

## Claims

1. Method for producing a hand-held medical instrument (2) which has two, preferably identically constructed, grip elements (14, 16) which can be pivoted relative to each other and a spring element (28) which is preferably of leaf-spring construction, having the method steps:
- providing the two grip elements (14, 16) each with a pivot-pin receiver eyelet formed thereon, at their one end portion and an articulation eyelet (36) for the spring element (28) formed thereon, and preferably slotted in its central portion
- providing the spring element (28) with pivot pins (44) arranged at each spring end or preferably formed integrally with the spring element (28),
- inserting the pivot pins (44) of the spring element (28) into the articulation eyelets (36) of the grip elements (14, 16) not yet pivotally coupled to each other, and
- bringing together the grip elements (14, 16) in the region of their pivot pin receiver eyelets and pivotally coupling the grip elements (14, 16) by inserting a preferably rivet-shaped or screw-shaped pivot pin (9) into the overlapping pivot pin receiver eyelets.

## Revendications

1. Procédé pour fabriquer un instrument manuel médical (2), lequel présente deux éléments de préhension (14, 16) de préférence de structure identique, pivotants l'un par rapport à l'autre et un élément ressort (28), réalisé de préférence à la façon d'une conception de ressort à lames, avec les étapes de procédé :
- fourniture des deux éléments de préhension (14, 16) respectivement avec un œillet de réception de tourillon réalisé sur ceux-ci sur l'une des parties d'extrémité de celui-ci et un œillet d'articulation (36), réalisé sur celui-ci, de préférence fendu dans la partie centrale de celui-ci, pour l'élément ressort (28),
- fourniture de l'élément ressort (28) avec respectivement un tourillon (44) disposé sur chaque extrémité de ressort ou réalisé, de préférence sans discontinuité de matière, avec l'élément ressort (28),
- insertion des tourillons (44) de l'élément ressort (28) dans les œillets d'articulation (36) des éléments de préhension (14, 16) pas encore accouplés de manière pivotante l'un à l'autre et
- réunion des éléments de préhension (14, 16) dans la zone des œillets de réception de tourillon de ceux-ci et accouplement pivotant des éléments de préhension (14, 16) par introduction d'un tourillon (9), de préférence en forme de rivet ou de vis, dans les œillets de réception de tourillon pivotant se chevauchant.
